# EUROPEAN PATENT SPECIFICATION

(11) **EP 3 704 487 B1**
(45) Date of publication and mention of the grant of the patent: **01.09.2021**
(21) Application number: 18815946.1
(22) Date of filing: 30.10.2018
(51) Int. Cl.: G01N 33/543, C09D 11/52, C09D 11/037, C09D 11/322, C12Q 1/00

(54) **BIOSENSOR MANUFACTURE**
BIOSENSORHERSTELLUNG
FABRICATION DE BIOCAPTEUR

(30) Priority: 02.11.2017 EP 17199766
(43) Date of publication of application: 09.09.2020
(73) Proprietor: Elisha Systems Limited, Cheshire SK10 2XR (GB)
(72) Inventor: GIBSON, Timothy David, Wakefield Yorkshire WF3 4AA (GB); SHARP, Duncan William Mackenzie, Althorpe North Lincolnshire DN17 3HZ (GB)
(74) Representative: Hepworth Browne
(86) International application number: PCT/EP2018/079742
(87) International publication number: WO 2019/086473

(56) References cited:
- WO-A1-2014/096407
- US-A1- 2008 187 651
- WISITSORAAT A ET AL: "Graphene-PEDOT:PSS on screen printed carbon electrode for enzymatic biosen", JOURNAL OF ELECTROANALYTICAL CHEMISTRY, vol. 704, 19 July 2013 (2013-07-19), pages 208-213, XP028698114, ISSN: 1572-6657, DOI: 10.1016/J.JELECHEM.2013.07.012
- PHONGPHUT A ET AL: "A disposable amperometric biosensor based on inkjet-printed Au/PEDOT-PSS nanocomposite for triglyceride determination", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 178, 11 January 2013 (2013-01-11), pages 501-507, XP028993272, ISSN: 0925-4005, DOI: 10.1016/J.SNB.2013.01.012
- WONG ADEMAR ET AL: "Determination of piroxicam and nimesulide using an electrochemical sensor based on reduced graphene oxide and PEDOT:PSS", JOURNAL OF ELECTROANALYTICAL CHEMISTRY, ELSEVIER, AMSTERDAM, NL, vol. 799, 29 June 2017 (2017-06-29), pages 547-555, XP085195871, ISSN: 1572-6657, DOI: 10.1016/J.JELECHEM.2017.06.055
- SAURABH KUMAR ET AL: "Conducting paper based sensor for cancer biomarker detection", JOURNAL OF PHYSICS: CONFERENCE SERIES, INSTITUTE OF PHYSICS PUBLISHING, BRISTOL, GB, vol. 704, no. 1, 19 April 2016 (2016-04-19), page 12010, XP020300849, ISSN: 1742-6596, DOI: 10.1088/1742-6596/704/1/012010 [retrieved on 2016-04-19]
- SAURABH KUMAR ET AL: "Reduced graphene oxide modified smart conducting paper for cancer biosensor", BIOSENSORS AND BIOELECTRONICS, vol. 73, 1 November 2015 (2015-11-01), pages 114-122, XP055457238, NL ISSN: 0956-5663, DOI: 10.1016/j.bios.2015.05.040
- SEEKAEW YOTSARAYUTH ET AL: "Low-cost and flexible printed graphene-PEDOT:PSS gas sensor for ammonia detec", ORGANIC ELECTRONICS, vol. 15, no. 11, 10 September 2014 (2014-09-10), pages 2971-2981, XP029076517, ISSN: 1566-1199, DOI: 10.1016/J.ORGEL.2014.08.044
- MICHAL WAGNER ET AL: "Synthesis and optimization of PEDOT:PSS based ink for printing nanoarrays using Dip-Pen Nanolithography", SYNTHETIC METALS, vol. 181, 1 October 2013 (2013-10-01), pages 64-71, XP055457242, CH ISSN: 0379-6779, DOI: 10.1016/j.synthmet.2013.08.012
- KISHORE KUMAR JAGADEESAN ET AL: "Application of conducting paper for selective detection of troponin", ELECTROCHEMISTRY COMMUNICATIONS, ELSEVIER, AMSTERDAM, NL, vol. 20, 27 March 2012 (2012-03-27), pages 71-74, XP028518351, ISSN: 1388-2481, DOI: 10.1016/J.ELECOM.2012.03.041 [retrieved on 2012-04-04]
- SUBBIAH JEGADESAN ET AL: "Direct Electrochemical Nanopatterning of Polycarbazole Monomer and Precursor Polymer Films: Ambient Formation of Thermally Stable Conducting Nanopatterns", LANGMUIR, vol. 22, no. 2, 1 January 2006 (2006-01-01), pages 780-786, XP055457247, US ISSN: 0743-7463, DOI: 10.1021/la0517686

## Description

This invention relates to manufacture of biosensors, particularly but not exclusively for automated or robotic manufacture of electrochemical sensors which incorporate biological materials.

Kumar, S. et al, Biosensors and Bioelectronics, 73, (2015), 114-122 discloses a reduced graphene oxide modified conducting paper for a cancer biosensor, in which conducting nanoparticles are deposited onto a porous paper substrate followed by physical absorption of anti-CEA onto the surface.

According to the present invention a method of manufacture of an electrochemical sensor precursor comprises the steps of:
preparing an adherent electrically conductive biocomposite comprising a mixture of a colloidal suspension in an aqueous liquid phase; the mixture including:
   polycationic conductive polymer particles;
   an anionic polyelectrolyte;
   or a composite of a polycationic conducting polymer and an anionic polyelectrolyte
   a biological affinity agent; and
   an optional buffer;
   applying the mixture to a region of an electroconductive surface; and removing water to allow adhesion of the biocomposite to the region of the surface.

The biocomposite mixture may form a non-catalytic coating.

It has been unexpectedly found that biocompositse of this invention may adhere to suitable substrates, for example gold substrates. The biocomposites may not comprise covalent linkages between the biological agent and the polymer particles. The biocomposition mixture may be used as an ink.

The biosensors may be used in impedimetric assay systems using non-catalytic biological agents. These may be contrasted with enzymic or other catalytic reagents such as are used in voltammetric or amperometric systems.

The affinity agents may be selected from Affimers and antibodies. Affimers are engineered non-antibody binding proteins which may mimic molecular recognition characteristics of antibodies.

The systems of the present invention may be used to observe or measure binding of a target analyte to a biological affinity agent incorporated into the deposited biocomposition.

The present invention has the advantage of providing an all-in-one biocomposite which may be applied and adhere to an electrode substrate in a single step. This greatly simplifies manufacture of a biosensor in comparison to all previously disclosed systems.

A single liquid biocomposite may be provided, which may contain all of the required ingredients. This may facilitate pre-association of the biological and non-biological components to provide a stable, active composition that may be transported, stored and deposited as required to provide a stable bioactive film capable of specific recognition to give a signal reporting the presence of one or more target analytes.

The biocomposites may be transparent, facilitating use in photochromic devices or opaque. Both transparent and opaque biocomposites are effective in electrochemical devices, such as biosensors.

Examples of target analytes
- Vascular biomarkers:: LOX1
- Cancer biomarkers:: PSA, CA125, Bladder cancer FGFR3 and Colon Cancer CEA
- Stroke biomarkers:: S-100 protein, Neuron Specific Enolase.
- Multiple Sclorosis biomarker:: Myelin Basic Protein.
- Cardiac biomarkers:: Myoglobin, Troponin I, Haemoglobin, Atrial natriuretic peptide (ANP)

Inflammation / Infection Biomarker C-Reactive Protein (CRP)
- Antibiotics and Drugs:: Fluoroquinolines, sulphonamides and digoxin.
- Herbicides: Atrazine
- Bacteria:: Staphylococcus aureus, MRSA Clostridium difficile Group A Streptococcus (Streptococcus pyogenes) Serogroup B Meningococcus (colominic acid) E coli Neisseria gonorrhoea Gram -ve and Gram +ve Bacterial panels
- Bacterial biomarker: PBP2a
- Other Proteins:: human Chorionic Gonadotropin (hCG) human serum albumin (HSA)
- Virus's: Adenovirus 5

Examples of the required components are set out below, but not in any limitative sense.

### 1) Polycationic conductive polymer particles

The polycationic conducting polymer may comprise particles having a dimension of about 5 to 750nm, optionally about 10 to about 500nm, optionally about 30 to about 400nm, optionally about 70 to about 400nm. The particles may comprise nanoparticles, nanotubes or mixtures thereof.

The polycationic conductive polymer may be selected from the group consisting of: polythiophenes and related polymers e.g. poly(3-methylthiophene), Poly(3-butylthiophene-2,5-diyl), poly(3-hexylthiophene) and the different regioregular polythiophenes e.g. regioregular poly(3-butylthiophene-2,5-diyl) and poly(3-hexylthiophene). Poly(3,4-ethylenedioxythiophene) (PEDOT) and related polymers e.g. poly (3,4-propylenedioxythiophene (PProDOT), and mixtures thereof. Alternative conductive polymers include polypyrrole and substituents thereof for example, poly (pyrrole-3-carboxilic acid), poly(1-cyanoethyl)pyrrole and poly(n-methyl)pyrrole), poly(polycarbazole and substituents thereof.

Alternative conductive polymers may be selected from the group consisting of polyaniline (PANI), and substituted polyanilines for example poly(2-aminobenzylamine), poly(2-aminobenzoic acid) and poly(2-methyl-aniline) and mixtures thereof.

Advantageous polycationic conductive polymers are selected from sulphur-containing polyheterocyclic compounds. Sulphur-containing cyclic coating compounds may form strong bonds to gold, platinum or other noble metal electrodes, with polythiophenes being some of the strongest.

### 2) Anionic polyelectrolyte

The anionic polyelectrolyte may be selected from the group consisting of: polystyrene sulphonic acid (PSS), alginic acid, poly(acrylic acid), poly(vinyl sulphonic acid), dextran sulphate, heparin, and multi-anionic dyestuffs including direct red 80, direct blue 15, direct blue 14, direct blue 1, direct blue 6, acid blue 29, acid black 1, acid red 27, acid red 113 and mixtures thereof.

Exemplary anionic polyelectrolytes have 20 or more acid groups and may have a molecular weight greater than 20,000, for example 40,000 or greater.

### 3) Pre-existing composites of polycationic conducting polymers and anionic polyelectrolytes

Pre-existing composites of Poly(3,4-ethylenedioxythiophene) PEDOT and Poly(styrenesulphonic acid) PSS are commercially available, for example from Bayer AG under the trade mark Baytron. A combination of PSS which is water soluble, with the insoluble PEDOT may give a material which acts as a solution but which comprises a dispersion of nanoparticles having various Redox states dependent on pH and other conditions. The measurement of particle size in 50mM MOPS buffer at pH 7.05 provided particles of two sizes at about 38nm and about 825nm, although the larger particles could be aggregates. Dimensions of about 70nm to about 400nm have been obtained. Commercial PEDOT/PSS pre-existing composites for example as available from Sigma Aldrich, comprise 3 to 4 wt% in water at pH 5. Adding 50mM, pH 7.5 MOPS buffer may effectively adjust the pH of the aqueous composite to 7. This may be a key step to incorporate active biological molecules.

Alternative pre-existing composites of polycationic conducting polymers and anionic polyelectrolytes include: polyaniline (PANI) nanoparticles and PSS, PANI/direct blue 15, PANI/direct red 80, PANI/acid red 27, Poly(2-methyl aniline) nanoparticles and PSS, Poly(2-methyl aniline)/direct blue 15, Poly(2-methyl aniline)/direct red 80, Poly(2-methyl aniline)/acid red 27, Polypyrrole nanoparticles and PSS, Polypyrrole/direct blue 15, Polypyrrole/direct red 80 and Polypyrrole/acid red 27.

Pre-existing composites of polythiophenes, e.g. PEDOT and PSS have an advantage of being very stable in electrochemical applications and may be used for more than 100 cycles without degradation.

### 4) Biological affinity agent

The biological affinity agent may be an antibody (polyclonal or monoclonal), antibody fragments such as F(ab) fragments, F(ab)₂ fragments or nanobodies (cloned Fv fragments), a binding protein such as cellulose binding protein, a nucleic acid such as DNE, RNA or mixtures thereof. In advantageous embodiments the affinity agent is an antibody biomimic such as an Affimer, Darpin or other conserved scaffold protein.

### 5) Optional buffer

Buffers control the pH of aqueous solutions and form environments where biological affinity agents are most stable. The addition of a buffer into the biocomposite mixture is optional. Buffers include mixtures of phosphate salts e.g. disodium hydrogen phosphate and sodium dihydrogen phosphate, citric acid and sodium citrate, sodium tetraborate and boric acid. Advantageous buffers include those designated as 'Good' buffers, which are which are zwitterionic compounds, having a pKa value between 6 and 8. 'Good' buffers have low toxicity, are highly watersoluble, stable against pH changes, have hydrolytic and enzymatic stability and have less effects on biochemical reactions. They were first prepared by N.E.Good et al in 1966. Examples of 'Good' buffers include 3-(N-Morpholino)propanesulfonic acid or MOPS, 1,4-Piperazinediethanesulfonic acid or PIPES, 4-(2-Hydroxyethyl)piperazine-1-ethanesulfonic acid or HEPES and N-[Tris(hydroxymethyl)methyl]-3-aminopropanesulfonic acid or TAPS.

Addition of buffers into the biocomposite mixture may improve the adhesion of the biocomposite coating formed.

The addition of a biological affinity agent, for example an antibody protein to a PSS solution forms a complex due to electrostatic interaction between the protein and the PSS molecules to give a protein-polyelectrolyte complex. Addition of an antibody protein to a PEDOT:PSS nanoparticle suspension in an aqueous solution forms a complex mixture of the protein and the anionic (PSS) and cationic polymer (PEDOT) nanoparticles. It has been found that addition of such a protein/PEDOT/PSS nanoparticle biocomposite to an electro conductive surface, for example a screen printed gold electrode, has the unexpected result that the biocomposite adheres strongly to the surface and is not removed by washing with distilled water, buffer solutions or buffered non-ionic detergents such Tween 20 at a concentration of 0.1v/v%. Furthermore addition of multiple layers of the protein/PEDOT/PSS nanoparticle biocomposite to a surface is possible to allow building up of a strongly adherent coating.

The biocomposition may be a nanocomposite material consisting of an aggregate or covalent complex of any of the above with a polymer molecule or nanoparticle (e.g. a gold nanoparticle-Affimer complex or gold nanoparticle-antibody complex or a dendrimer nanocomposite).

The method may further comprise the steps of application of one or more aliquots of the aqueous biocomposite to a selected region of the surface of an electrode, allowing the biocomposite to dry, forming a coating on the electrode. The step of adding the aqueous composite may be repeated as necessary to build up a layer on the surface.

The biocomposite may be applied to the substrate by drop casting, printing, spraying, using an air jet or other deposition method.

The electroconductive surface may be a region of an electroconductive electrode.

Herein disclosed is also an electrochemical sensor precursor comprises an electroconductive surface comprising a plurality of electrodes, one electrode including a layer of an electrically conductive composite comprising polycationic conductive polymer particles, an anionic polyelectrolyte and a biological affinity agent.

The biological affinity agent is a non-catalytic agent as described above.

The electroconductive surface which may comprise an electrode may be composed of screen printed or ink jet printed carbon or noble metal, for example, gold or platinum.

Photolithographically or sputtered gold or platinum electrodes may be employed. However the use of photolithographically or sputtered gold electrodes may not be preferred on account of their high cost.

Use of a manufacture method in accordance with the present invention confers numerous advantages. The resulting biosensors have been found to function well in both Faradaic and non-Faradaic buffers, for example ferri/ferrocyanide buffer or in non-conductive buffers such as phosphate buffered saline (PBS). This unexpectedly provides the advantage that it allows an analyte solution to be added directly without modifying using a buffer or other additive solution. Also electropolymerization of the polymer coating onto the conductive surface is avoided, facilitating large scale robotic manufacture of arrays of the biosensors.

The biosensors may be employed in apparatus using AC impedance interrogation.

Alternatively or in addition or other methods to interrogate the non-catalytic coatings formed may include cyclic voltammetry, differential pulse voltammetry, chronopotentiometry and open circuit potentiometry.

The invention is further described by means of examples but not in any limitative sense with reference to the accompanying drawings of which:
Figure 1 shows a calibration curve for a PEDOT:PSS Myoglobin Affimer biocomposite biosensor;
Figure 2 shows a calibration curve for a PEDOT:PSS Troponin-I biocomposite biosensor;
Figure 3 shows the degree of luminescence of a PEDOT:PSS biocomposite biosensor;
Figure 4 shows a calibration curve for a PEDOT:PSS Bacteria biocomposite biosensor;
Figure 5 shows a calibration curve for a PEDOT:PSS PBP2a biocomposite biosensor.

Example 1 - Preparation of PEDOT: PSS Affimer biocomposite and consequent biosensor for Myoglobin measurement.

An Affimer biocomposite was prepared by the following method:

A 1 in 10 dilution of PEDOT:PSS nanoparticles (Sigma Aldrich catalogue number 655201, high conductivity grade 3-4%w/v dispersion in water) into deionised water was first made.

Then 10µl of Anti-Human Myglobin Affimer (1mg per ml in Affimer elution buffer pH 8.0 containing phosphate buffer, sodium chloride, imidazole and glycerol) was added to 40µl of the 1/10 diluted PEDOT:PSS suspension, to give an Affimer/PEDOT:PSS biocomposite containing 200µg per ml Affimer, designated as - specific Affimer complex or SAC.

A second Affimer biocomposite was prepared using Anti-ANP Affimer (1mg per ml in 100mM PBS buffer pH 7.1) adding to 40µl of the 1/10 diluted PEDOT:PSS suspension, to give an Affimer/PEDOT:PSS biocomposite containing 200µg per ml Affimer, designated as - non-specific Affimer complex or NSAC.

1.0µl of SAC was deposited on the upper surface of working electrode 2 (WE2) of a dual gold electrode (DropSens X222OAT) and 1.0µl or NSAC was deposited on the upper surface of working electrode 1 (WE1) of a dual gold electrode, then the deposited biocomposites were incubated at room temperature for 10 minutes and then dried on a hot plate set at 40°C for a further 10 minutes. This procedure gave a thin adherent layer of Affimer/PEDOT:PSS biocomposite adhering strongly to the surface of the gold electrodes.

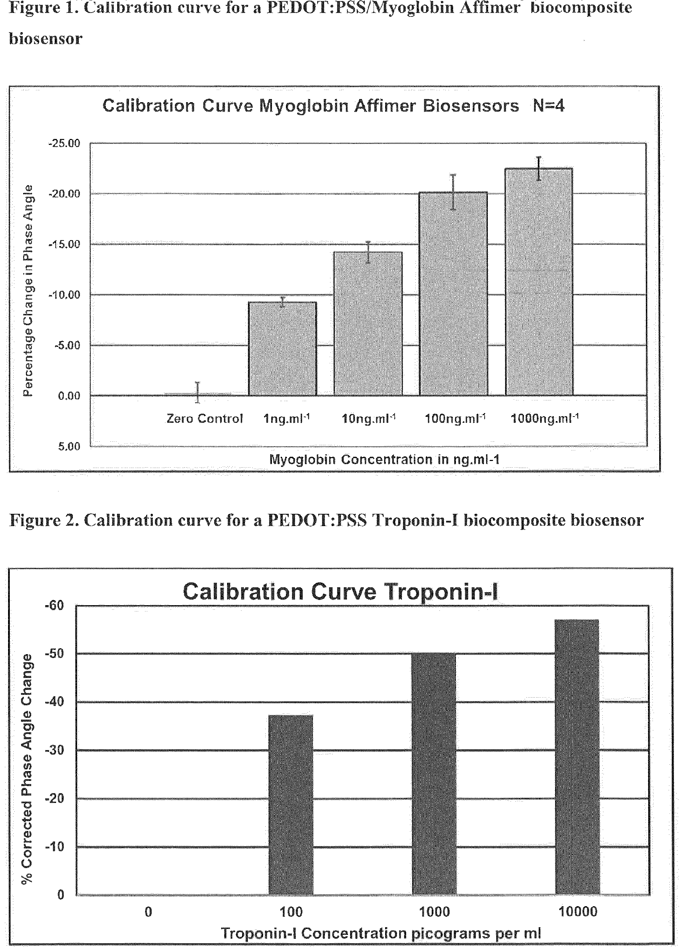

Incubation of solutions of Human Myoglobin onto both electrode surfaces for 10 minutes resulted in measurable changes to the AC impedance when they were scanned between 2500Hz down to 0.25Hz in ferri/ferrocyanide buffer (Faradaic buffer), indicating affinity binding took place. Any non-specific binding that occurred was corrected for by the NSAC on WE1 by subtracting the non-specific signal obtained from the signal from WE2. The differential signal (WE2-WE1) was a measure of the concentration of Human Myoglobin. A concentration dependant calibration curve is depicted in Figure 1, where the results are normalized to a differential percentage impedance change. Four Affimer biocomposite biosensors gave good reproducibility as indicated by the error bars on the graph in figure 1.

Example 2 - Preparation of PEDOT: PSS biocomposite and consequent biosensor for Troponin-I measurement.

A biocomposite was prepared by the following method:
A 1 in 10 dilution of PEDOT:PSS nanoparticles (Sigma Aldrich catalogue number 655201, high conductivity grade 3-4%w/v dispersion in water) into 50mM MOPS buffer pH 7.05 was first made.
Then 10µl of polyclonal anti-troponin-I (1mg per ml in 50mM MOPS buffer pH 7.05) was added to 40µl of the 1/10 diluted PEDOT:PSS suspension, to give an antibody/PEDOT:PSS biocomposite containing 200µg per ml antibody, designated as - specific antibody complex or SAC.

A second antibody biocomposite was prepared using polyclonal anti-digoxin (2mg per ml in 100mM PBS buffer pH 7.1) adding to 90µl of the 1/10 diluted PEDOT:PSS suspension, to give an antibody/PEDOT:PSS biocomposite containing 200µg per ml antibody, designated as - non-specific antibody complex or NSAC.

0.4µl of SAC was deposited on the upper surface of working electrode 2 (WE2) of a dual gold electrode (DropSens X222OAT) and 0.4µl or NSAC was deposited on the upper surface of working electrode 1 (WE1) of a dual gold electrode, then the deposited suspensions were dried on a hot plate set at 37°C. This procedure was repeated 2 further times to give a thin layer of antibody/PEDOT:PSS biocomposite adhering strongly to the surface of the gold electrodes.

Incubation of solutions of Troponin-I onto both electrode surfaces for 5 minutes resulted in measurable changes to the AC impedance when they were scanned between 1000Hz down to 1Hz in PBS (non-Faradaic buffer), indicating affinity binding took place. Any non-specific binding that occurred was corrected for by the NSAC on WE1 by subtracting the non-specific signal obtained from the signal from WE2. The differential signal (WE2-WE1) was a measure of the concentration of Troponin-I. A concentration dependant calibration curve is depicted in Figure 2.

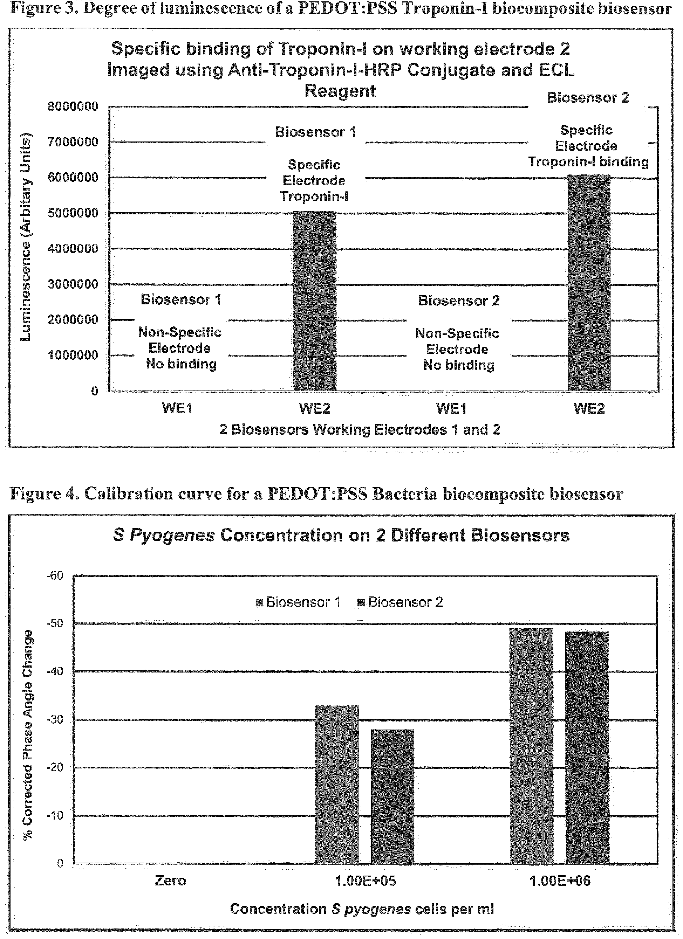

Example 3 - Demonstration of adherence of protein to the electrode surface Strong adherence of protein:PEDOT:PSS nanoparticle biocomposites to electrode surfaces and specific binding capabilities was demonstrated using an optical imaging technique. The electrode surfaces were prepared using a polyclonal Anti-Troponin-I added to PEDOT:PSS nanoparticles in MOPS buffer (45mM, pH 7.05) to give an active biocomposite. Three aliquots of 0.2µl were drop cast onto the electrode surfaces and dried at 37°C for 2-3 minutes between aliquots. The control electrode was prepared using polyclonal Anti-Digoxin in exactly the same manner. The biosensors thus prepared were incubated with analyte (Troponin-I) and then washed with buffered 0.1% v/v Tween 20 to remove any non-specific bound analyte. Then an Anti-Troponin-HRP conjugate was used to label the bound analyte, washed again with buffered 0.1% v/v Tween 20 to remove any non-specific bound conjugate. Pierce ECL reagent was then added and the electrodes imaged in a GeneSys imager. The surfaces luminesced due to the bound HRP label. A higher degree of luminescence indicated bound analyte, Figure 3.

Example 4 - Preparation of PEDOT: PSS biocomposite and consequent biosensor for *Streptococcus pyogenes* measurement.

A biocomposite was prepared by the following method:
A 1 in 10 dilution of PEDOT:PSS nanoparticles (Sigma Aldrich catalogue number 655201, high conductivity grade 3-4%w/v dispersion in water) into 50mM MOPS buffer pH 7.05 was first made.

Then 10µl of polyclonal anti- *Streptococcus pyogenes* (1mg per ml in 50mM MOPS buffer pH 7.05) was added to 40µl of the 1/10 diluted PEDOT:PSS suspension, to give an antibody/PEDOT:PSS biocomposite containing 200µg per ml antibody, designated as - specific antibody complex or SAC.

A second antibody biocomposite was prepared using polyclonal anti-digoxin (2mg per ml in 100mM PBS buffer pH 7.1) adding to 90µl of the 1/10 diluted PEDOT:PSS suspension, to give an antibody/PEDOT:PSS biocomposite containing 200µg per ml antibody, designated as - non-specific antibody complex or NSAC.

0.2µl of 1 in 10 dilution of PEDOT:PSS nanoparticles was deposited on the upper surface of both working electrodes (WE1 and WE2) of a dual gold electrode (DropSens X222OAT) and dried on a hot plate set at 37°C. When dry 1 µl of NSAC was deposited on the upper surface of working electrode 1 (WE1) and 1 µl of SAC was deposited on the upper surface of working electrode 2 (WE2) of a dual gold electrode, then the deposited suspensions were dried on a hot plate set at 37°C. This 2 stage procedure gave a thin layer of antibody/PEDOT:PSS biocomposite adhering strongly to the surface of the gold electrodes.

Incubation of suspensions of *Streptococcus pyogenes* onto both electrode surfaces for 15 minutes resulted in measurable changes to the AC impedance when they were scanned between 1000Hz down to 1Hz in ferri/ferrocyanide buffer (Faradaic buffer), indicating affinity binding took place. Any non-specific binding that occurred was corrected for by the NSAC on WE1 by subtracting the non-specific signal obtained from the signal from WE2. The differential signal (WE2-WE1) was a measure of the concentration of *Streptococcus pyogenes.* A concentration dependant calibration curve is depicted in Figure 4.

Example 5 - Preparation of PEDOT: PSS biocomposite and consequent biosensor for Penicillin Binding Protein 2a (PBP2a) measurement. PBP2a is a biomarker for MRSA.

A biocomposite was prepared by the following method:
A 1 in 10 dilution of PEDOT:PSS nanoparticles (Sigma Aldrich catalogue number 655201, high conductivity grade 3-4%w/v dispersion in water) into 50mM MOPS buffer pH 7.05 was first made.

Then 10µl of polyclonal anti-PBP2aI (1mg per ml in 50mM MOPS buffer pH 7.05) was added to 40µl of the 1/10 diluted PEDOT:PSS suspension, to give an antibody/PEDOT:PSS biocomposite containing 200µg per ml antibody, designated as - specific antibody complex or SAC.

A second antibody biocomposite was prepared using polyclonal anti-digoxin (2mg per ml in 100mM PBS buffer pH 7.1) adding to 90µl of the 1/10 diluted PEDOT:PSS suspension, to give an antibody/PEDOT:PSS biocomposite containing 200µg per ml antibody, designated as - non-specific antibody complex or NSAC.

0.4µl of SAC was deposited on the upper surface of working electrode 2 (WE2) of a dual gold electrode (DropSens X222OAT) and 0.4µl or NSAC was deposited on the upper surface of working electrode 1 (WE1) of a dual gold electrode, then the deposited suspensions were dried on a hot plate set at 37°C. This procedure was repeated 2 further times to give a thin layer of antibody/PEDOT:PSS biocomposite adhering strongly to the surface of the gold electrodes.

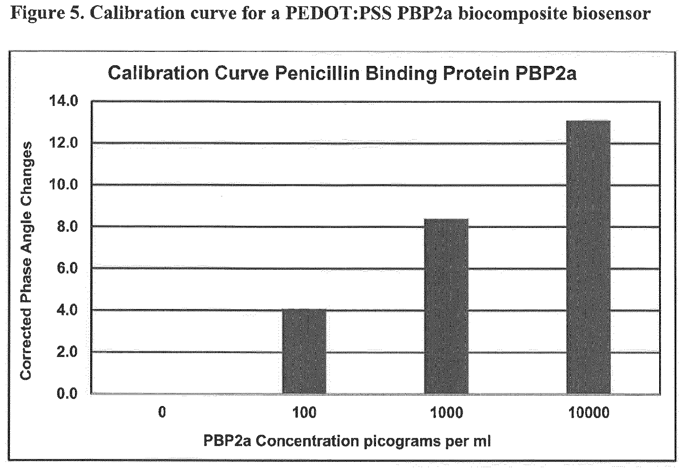

Incubation of solutions of PBP2a onto both electrode surfaces for 5 minutes resulted in measurable changes to the AC impedance when they were scanned between 1000Hz down to 1Hz in ferri/ferrocyanide buffer (Faradaic buffer), indicating affinity binding took place. Any non-specific binding that occurred was corrected for by the NSAC on WE1 by subtracting the non-specific signal obtained from the signal from WE2. The differential signal (WE2-WE1) was a measure of the concentration of PBP2a. A concentration dependant calibration curve is depicted in Figure 5.

## Claims

1. A method of manufacture of an electrochemical sensor biocomposite precursor comprising the steps of:
preparing an adherent electrically conductive biocomposite comprising a mixture of a colloidal suspension in an aqueous liquid phase; the mixture including:
polycationic conductive polymer particles;
an anionic polyelectrolyte;
or pre-existing composites of polycationic conducting polymers and anionic polyelectrolytes
a biological affinity agent; and
an optional buffer;
applying the mixture to a region of an electroconductive surface; and
removing water to allow adhesion of the biocomposite to the region of the surface.

2. A method as claimed in claim 1, wherein the biocomposite is non-catalytic.

3. A method as claimed in claim 1 or 2, wherein the agent is an Affimer.

4. A method as claimed in claim 1 or 2 wherein the agent is an antibody.

5. A method as claimed in claim 1 or 2, wherein the polymer comprises particles having a dimension of 5 to 750nm.

6. A method as claimed in claim 3, wherein the particles have a dimension of 10 to 500nm.

7. A method as claimed in claim 4, wherein the particles have a dimension of 30 to 400nm.

8. A method as claimed in claim 5, wherein the particles have a dimension of 70 to 400nm.

9. A method as claimed in any preceding claim, wherein the particles comprise nanoparticles, nanotubes or mixtures thereof.

10. A method as claimed in any preceding claim, wherein the conductive polymer is related from the group consisting of: poly (3,4-ethylenedioxythiophene) (PEDOT) and related polymers e.g. poly (3,4-propylenedioxythiophene (PProDOT), polypyrrole and substituted polypyrroles, poly(pyrrole-3-carboxylic acid), poly(1-cyanoethyl)pyrrole and poly(n-methyl)pyrrole), and polycarbazole and substituted polycarbazoles.

11. A method as claimed in any of claims 1 to 7, wherein the conductive polymer is selected from the group consisting of: polyaniline (PANI) and mixtures thereof; substituted polyanilines for example, poly(2-aminobenzylamine), poly(2-aminobenzoic acid) and poly(2-methyl-aniline).

12. A method as claimed in any preceding claim, wherein the anionic polyelectrolyte is selected from the group consisting of:
polystyrene sulphonic acid (PSS), alginic acid and mixtures thereof;
and multi-anionic dyestuffs including: direct red 80, direct blue 15, direct blue 14, direct blue 1, direct blue 6, acid blue 29, acid black 1, acid red 27, acid red 113 and mixtures thereof.

13. A method as claimed in any of claims 2 to 10, wherein the biological affinity agent is selected from the group consisting of:
a polyclonal antibody or monoclonal antibody, antibody fragments, for example, F(ab) fragments, F(ab)2 fragments or nanobodies (cloned Fv fragments), a binding protein, for example: cellulose binding protein, a nucleic acid such as DNE, RNA or composites thereof, an antibody biomimic, for example, Affimers, Darpins or other conserved scaffold proteins.

14. A method as claimed in any preceding claim, comprising the steps of application of aliquots of the aqueous biocomposite to a selected region of the surface of an electrode, allowing the composite to dry, forming a coating on the electrode.

15. A method as claimed in claim 12, wherein the biocomposite is applied by drop casting, robotically controlled liquid deposition, printing or spraying.

## Patentansprüche

1. Verfahren zur Herstellung einer Biokompositvorstufe eines elektrochemischen Sensors, das folgende Schritte umfasst:
- Herstellen eines adhärenten elektrisch leitfähigen Biokomposits, umfassend eine Mischung einer kolloidalen Suspension in einer wässrigen, flüssigen Phase; wobei die Mischung Folgendes enthält:
- polykationische, leitfähige Polymerteilchen;
- einen anionischen Polyelektrolyten;
- oder bereits vorhandene Komposite aus polykationischen, leitfähigen Polymeren und anionischen Polyelektrolyten,
- ein biologisches Affinitätsmittel; und
- einen optionalen Puffer;
- Aufbringen der Mischung auf einen Bereich einer elektrisch leitenden Oberfläche; und
- Entfernen von Wasser, um das Anhaften des Biokomposits an den Bereich der Oberfläche zu ermöglichen.

2. Verfahren nach Anspruch 1,
wobei das Biokomposit nicht katalytisch ist.

3. Verfahren nach Anspruch 1 oder 2,
wobei das Mittel ein Affimer ist.

4. Verfahren nach Anspruch 1 oder 2,
wobei das Mittel ein Antikörper ist.

5. Verfahren nach Anspruch 1 oder 2,
wobei das Polymer Teilchen mit Abmessungen von 5 nm bis 750 nm umfasst.

6. Verfahren nach Anspruch 3,
wobei die Teilchen Abmessungen von 10 nm bis 500 nm aufweisen.

7. Verfahren nach Anspruch 4,
wobei die Teilchen eine Abmessungen von 30 nm bis 400 nm aufweisen.

8. Verfahren nach Anspruch 5,
wobei die Teilchen Abmessungen von 70 nm bis 400 nm aufweisen.

9. Verfahren nach einem vorhergehenden Anspruch,
wobei die Teilchen Nanopartikel, Nanoröhren oder Mischungen davon aufweisen.

10. Verfahren nach einem vorhergehenden Anspruch,
wobei das leitfähige Polymer mit der Gruppe verknüpft ist, die aus folgenden Substanzen besteht: Poly(3,4-ethylendioxythiophen) (PEDOT) und verwandten Polymeren, z.B. Poly(3,4-propylendioxythiophen (PProDOT), Polypyrrol und substituierten Polypyrrolen, Poly(pyrrol-3-carbonsäure), Poly(1-cyanoethyl)pyrrol und Poly(n-methyl)pyrrol), und Polycarbazol und substituierten Polycarbazolen.

11. Verfahren nach einem der Ansprüche 1 bis 7,
wobei das leitfähige Polymer ausgewählt ist aus der Gruppe, die aus folgenden Substanzen besteht: Polyanilin (PANI) und Mischungen davon; substituierten Polyanilinen, zum Beispiel Poly(2-aminobenzylamin), Poly(2-aminobenzoesäure) und Poly(2-methylanilin).

12. Verfahren nach einem vorhergehenden Anspruch,
wobei der anionische Polyelektrolyt ausgewählt ist aus der Gruppe, die aus folgenden Substanzen besteht:
- Polystyrolsulfonsäure (PSS), Alginsäure und Mischungen davon;
- und multianionischen Farbstoffen, einschließlich: Direct Red 80, Direct Blue 15, Direct Blue 14, Direct Blue 1, Direct Blue 6, Acid Blue 29, Acid black 1, Acid Red 27, Acid Red 113 und Mischungen davon.

13. Verfahren nach einem der Ansprüche 2 bis 10,
wobei das biologische Affinitätsmittel ausgewählt ist aus der Gruppe, die aus folgenden Substanzen besteht:
- einem polyklonalen Antikörper oder monoklonalen Antikörper, Antikörperfragmenten, zum Beispiel: F(ab)-Fragmenten, F(ab)2-Fragmenten oder Nanobodies (klonierten Fv-Fragmenten), einem Bindungsprotein, zum Beispiel: Cellulose-Bindungsprotein, einer Nucleinsäure, wie z.B. DNE, RNA oder Kompositen davon, einem Antikörper-Biomimetikum, zum Beispiel: Affimeren, Darpins oder anderen konservierten Gerüstproteinen.

14. Verfahren nach einem vorhergehenden Anspruch,
umfassend die folgenden Schritte:
- Aufbringen von Aliquoten des wässrigen Biokomposits auf einen ausgewählten Bereich der Oberfläche einer Elektrode, und
- Trocknenlassen des Komposits unter Bildung einer Beschichtung auf der Elektrode.

15. Verfahren nach Anspruch 12,
wobei das Biokomposit durch Tropfgießen, robotergesteuerte Flüssigkeitsabscheidung, Drucken oder Sprühen aufgebracht wird.

## Revendications

1. Procédé de fabrication d'un précurseur biocomposite de capteur électrochimique comprenant les étapes de :
préparation d'un biocomposite adhérent à conduction électrique comprenant un mélange d'une suspension colloïdale dans une phase aqueuse liquide ;
le mélange comportant :
des particules de polymère polycationique à conduction électrique ;
un polyélectrolyte anionique ;
ou des composites préexistants de polymères polycationiques à conduction électrique et des polyélectrolytes anioniques ;
un agent à affinité biologique ; et
un tampon optionnel ;
l'application du mélange à une région d'une surface à conduction électrique ; et
l'enlèvement de l'eau afin de permettre l'adhésion du biocomposite à la région de la surface.

2. Procédé selon la revendication 1, selon lequel le biocomposite est non-catalytique.

3. Procédé selon la revendication 1 ou la revendication 2, selon lequel l'agent est un Affimer.

4. Procédé selon la revendication 1 ou la revendication 2, selon lequel l'agent est un anticorps.

5. Procédé selon la revendication 1 ou la revendication 2, selon lequel le polymère comprend des particules présentant une dimension de 5 à 750 nm.

6. Procédé selon la revendication 3, selon lequel les particules présentent une dimension de 10 à 500 nm.

7. Procédé selon la revendication 4, selon lequel les particules présentent une dimension de 30 à 400 nm.

8. Procédé selon la revendication 5, selon lequel les particules présentent une dimension de 70 à 400 nm.

9. Procédé selon l'une quelconque des revendications précédentes, selon lequel les particules comprennent des nanoparticules, nanotubes ou des mélanges de ceux-ci.

10. Procédé selon l'une quelconque des revendications précédentes, selon lequel le polymère à conduction électrique est apparenté au groupe consistant en : le poly-(3,4-éthylènedioxythiophène) (PEDOT) et des polymères apparentés, par exemple, le poly-(3,4-propylènedioxythiophène (PProDOT), le polypyrrole et les poly-pyrroles substitués, l'acide poly(pyrrole-3-carboxylique), le poly(1-cyanoéthyl)pyrrole et le poly(n-méthyl)pyrrole), et le polycarbazole et les polycarbazoles substitués.

11. Procédé selon l'une quelconque des revendications 1 à 7, selon lequel le polymère à conduction électrique est choisi dans le groupe consistant en :
la polyaniline (PANI) et des mélanges de celle-ci ;
les poly-anilines substituées, par exemple, la poly(2-aminobenzylamine), l'acide poly-(2-aminobenzoïque) et la poly-(2-méthyl-aniline) .

12. Procédé selon l'une quelconque des revendications précédentes, selon lequel le polyélectrolyte anionique est choisi dans le groupe consistant en :
l'acide sulfonique de polystyrène (PSS), l'acide alginique et des mélanges de ceux-ci ; et
des colorants multi-anioniques comportant : le rouge direct 80, le bleu direct 15, le bleu direct 14, le bleu direct 1, le bleu direct 6, le bleu acide 29, le noir acide 1, le rouge acide 27, le rouge acide 113 et des mélanges de ceux-ci.

13. Procédé selon l'une quelconque des revendications 2 à 10, selon lequel l'agent à affinité biologique est choisi dans le groupe consistant en :
un anticorps polyclonal ou monoclonal, des fragments d'anticorps, par exemple, des fragments F(ab), des fragments F(ab)2 ou des nanocorps (fragments Fv clonés), une protéine de liaison, par exemple : la protéine de liaison de la cellulose, un acide nucléique tel que le DNE, l'ARN ou des composites de ces derniers, un anticorps biomimétique, par exemple, des Affimers, des Darpins ou d'autres protéines d'échafaudage conservées.

14. Procédé selon l'une quelconque des revendications précédentes, comprenant les étapes d'application d'aliquotes du biocomposite aqueux à une région choisie de la surface d'une électrode, de séchage du composite, de formation d'un revêtement sur l'électrode.

15. Procédé selon la revendication 12, selon lequel le biocomposite est appliqué par moulage gravitaire, dépôt de liquide robotisé, impression ou pulvérisation.
